(19) Europäisches Patentamt
European Patent Office
Office européen des brevets

(11) Veröffentlichungsnummer: **0 203 495**
**B1**

(12) # EUROPÄISCHE PATENTSCHRIFT

(45) Veröffentlichungstag der Patentschrift: **01.03.89**

(51) Int. Cl.[4]: **C07D 333/32**

(21) Anmeldenummer: **86106821.1**

(22) Anmeldetag: **20.05.86**

(54) **Thiophenonderivate sowie Verfahren zu deren Herstellung.**

(30) Priorität: **21.05.85 CH 2158/85**

(43) Veröffentlichungstag der Anmeldung: **03.12.86 Patentblatt 86/49**

(45) Bekanntmachung des Hinweises auf die Patenterteilung: **01.03.89 Patentblatt 89/9**

(84) Benannte Vertragsstaaten: **AT BE CH DE FR GB IT LI LU NL SE**

(56) Entgegenhaltungen:
**EP-A- 0 130 025**

**TETRAHEDRON, Band 27, Nr. 16, August 1971, Seiten 3839-3851, Pergamon Press, GB; J.Z. MORTENSEN et al.: "THIOPHENE CHEMISTRY-XVIII* Preparation and tautomeric structures of some potential dihydroxythiophenes"**

(73) Patentinhaber: **LONZA AG, Gampel/Wallis(CH)**

(72) Erfinder: **Meul, Thomas, Dr., Balfrinstrasse 21, Visp (Kanton Wallis)(CH)**
Erfinder: **Tenud, Leander, Dr. Ing.-Chem., Balfrinstrasse 23, Visp (Kanton Wallis)(CH)**
Erfinder: **McGarrity, John, Dr., Rathausstrasse 5, Visp (Kanton Wallis)(CH)**

(74) Vertreter: **Weinhold, Peter, Dr. et al, Patentanwälte Dipl.-Ing. G. Dannenberg Dr. P. Weinhold Dr. D. Gudel Dipl.-Ing. S. Schubert Dr. P. Barz Siegfriedstrasse 8, D-8000 München 40(DE)**

**EP 0 203 495 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Thiophenonderivate, die einfach in Thiotetronsäure überzuführen sind.

Thiotetronsäure selber könnte als Zwischenprodukt zur Herstellung von (±)Thiolactomycin, einem Antibiotikum mit breitem Wirkungsspektrum, eine bedeutende Anwendungsmöglichkeit finden (Tetrahedron Letters Vol 25 No.46 pp 5243–5246, 1984).

Aus E. Benary, Chem. Berichte 46, 2103 (1913) ist bekannt, die Thiotetronsäure, ausgehend von Acetylthioglycoylchlorid, durch Reaktion mit Natriummalonester und anschliessendem Ringschluss und Wasserbehandlung herzustellen.

D.B. Macierewicz, Rocz. chem. 47, 1735 (1973) hat die Reaktion von E. Benary nachvollzogen und dabei Thiotetronsäure in einer Ausbeute von 30,3%, bezogen auf das eingesetzte Acetylthioglycoylchlorid, erhalten.

Eine andere Möglichkeit zeigt die Synthese von J.Z. Mortensen et al, Tetrahedron 27, 3839 (1971). Ausgehend von 2,4-Dibromthiophen, erhält er über 3 Stufen durch Umsetzung mit Butyllithium und t-Butylperbenzoat in einer Ausbeute von 46,2% die Thiotetronsäure.

Bei diesen herkömmlichen Synthesen sind nicht nur die Ausbeuten zu bescheiden, sondern auch die eingesetzten Edukte und Reagenzien lassen kaum an ein wirtschaftliches technisches Verfahren denken.

Ein weiteres Problem besteht in der Herstellung eines qualitativ hochwertigen Produktes, da Thiotetronsäure sowohl bei Umkristallisationen als auch in wässrigen Lösungen leicht unter Wasserabspaltung ein dimeres Kondensationsprodukt bildet.

Die Aufgabe der vorliegenden Erfindung war es daher, alternative Thiotetronsäurevorläufer zu finden, die technisch einfach herstellbar und aus dem Reaktionsgemisch isolierbar sind, in hoher Reinheit anfallen und einfach in Thiotetronsäure überführbar sind.

Erfindungsgemäss wird diese Aufgabe gelöst durch die neuen Thiophenonderivate der allgemeinen Formel

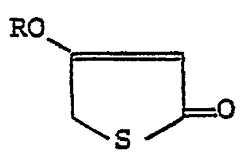

worin R eine $C_1$–$C_8$-Alkoxycarbonylgruppe, die unsubstituiert oder mit ($C_1$–$C_4$)-Alkylgruppe(n), Halogen-($C_1$–$C_4$)-alkylgruppe(n) oder Halogenatom(en) substituiert ist, eine Benzyloxycarbonylgruppe, die unsubstituiert oder mit Halogenatom(en), Nitrogruppe(n) oder ($C_1$–$C_4$)-Alkylgruppe(n) substituiert ist, oder eine p-Toluolsulfonylgruppe bedeutet. Gegenstand der Erfindung ist auch ein Verfahren zur Herstellung von solchen Thiophenonderivaten, das dadurch gekennzeichnet ist, daß 4-Chlor-4-chlormethyloxetan-2-on mit Schwefelwasserstoff in Gegenwart einer Base zur intermediär entstehenden Thiotetronsäure und diese anschliessend mit einem Chlorameisensäureester der allgemeinen Formel Cl-COOR, in der R die oben genannte Bedeutung besitzt, oder einem p-Toluolsulfonsäurechlorid zum Endprodukt umgesetzt wird.

Das 4-Chlor-4-chlormethyloxetan-2-on kann auf einfache Weise gemäss EP-Anmeldung 60 808 hergestellt und nach einer Flash-Destillation zur erfindungsgemässen Umsetzung eingesetzt werden.

Der Schwefelwasserstoff wird zweckmässig gasförmig eingesetzt.

Als geeignete Amine finden vorteilhaft primäre, sekundäre oder tertiäre Amine, Ammoniak oder auch Guanidine Anwendung.

Als besonders vorteilhaft werden die tertiären Amine, wie z.B. Triethylamin, angesehen.

Es ist ausserdem zweckmässig, die Reaktion in Lösungsmitteln durchzuführen. Eingesetzt werden gegenüber dem reaktiven Edukt inerte Lösungsmittel, wie halogenierte Kohlenwasserstoffe, Ether oder Carbonsäureester. Beispielsweise können Methylenchlorid, Chloroform, etherische Lösungsmittel, wie Tetrahydrofuran, angewendet werden.

Besonders bevorzugt wird aber Tetrahydrofuran oder Essigsäureethylester verwendet.

Die Edukte werden zweckmässig in einem Molverhältnis 4-Chlor-4-chlormethyloxetan-2-on zu Schwefelwasserstoff zu Amin zu Chlorameisensäureester/p-Toluolsulfonsäurechlorid von 1:1:2,8:0,8 bis 1:3:4:1, vorzugsweise von 1:2:2,8:0,8 bis 1:3:3,0:1, eingesetzt.

Es wird bevorzugt bei Temperaturen von –40 bis +20°C, besonders bevorzugt zwischen –20 bis –10°C, gearbeitet.

Zweckmässig wird so vorgegangen, dass das 4-Chlor-4-chlormethyloxetan-2-on, gelöst im inerten Lösungsmittel, mit Schwefelwasserstoff gesättigt wird und anschliessend in einem Zeitraum von 30 bis 120 Minuten das Amin zugegeben wird.

Nach der Aminzugabe kann, ohne die intermediär entstandene Thiotetronsäure abtrennen zu müssen, direkt, in Lösung, mit dem Chlorameisensäureester oder dem p-Toluolsulfonsäurechlorid zu den erfindungsgemässen Thiophenonderivaten umgesetzt werden.

Die Isolierung der Thiophenonderivate kann auf einfache Art durch Extraktion oder durch Umkristallisation erfolgen.

Es können auf diese Weise Ausbeuten von grösser als 70%, mit Gehalten von durchwegs grösser als 90%, in gewissen Fällen von grösser als 99%, erzielt werden.

Die Umsetzung der Thiophenonderivate in die Thiotetronsäuren wird basisch, zweckmässig in Gegenwart eines aliphatischen Amins oder $NH_3$, durchgeführt.

Nach zweckmässiger Aufarbeitung kann eine qualitativ hochwertige Thiotetronsäure, mit einem Gehalt von grösser als 95% erhalten werden.

Als besonders bevorzugte Verbindungen, die die eingangs genannten Vorteile in höchstem Mass erfüllen, gelten das

– 4-(Ethoxycarbonyloxy)-thiophen-2(5H)-on und das
– 4-(Methoxycarbonyloxy)-thiophen-2(5H)-on.

Beispiel 1

Eine Lösung von 15,9 g (0,1 Mol) 4-Chlor-4-chlormethyloxetan-2-on in 300 ml Tetrahydrofuran wurde auf –20°C abgekühlt und mit gasförmigem Schwefelwasserstoff gesättigt. Anschliessend gab man tropfenweise unter kräftigem Rühren innerhalb einer Stunde 20,4 g (0,2 Mol) Triethylamin zu. Man liess die Reaktionslösung auf Raumtemperatur erwärmen, rührte eine Stunde nach, filtrierte vom ausgefallenen Salz ab und dampfte das Lösungsmittel am Rotationsverdampfer zur Hälfte ab.

Diese Lösung wurde mit 9,8 g (0,09 Mol) Chlorameisensäureethylester versetzt, auf 0°C abgekühlt und tropfenweise mit 9,1 g (0,09 Mol) Triethylamin versetzt. Man filtrierte das ausgefallene Salz ab und engte die Reaktionslösung am Rotationsverdampfer ein. Der Rückstand wurde mit 400 ml Petrolether ausgekocht, die Lösung abdekantiert und auf –20°C abgekühlt. Die ausgefallenen Kristalle wurden abgenutscht.

Man erhielt 15,0 g des 4-(Ethoxycarbonyloxy)-thiophen-2(5H)-on in Form beige gefärbter Kristalle vom Smp. 52–54°C mit einem Gehalt von 99,2% (GC).

Dies entsprach 14,9 g 100%igem Produkt bzw. einer Ausbeute von 79,1%, bezogen auf eingesetztes Oxetanon.

$^1$H-NMR-Spektrum ($CDCl_3$, 300 MHz)
δ = 6,42 (t, $J_{3,5}$ = 1,3 Hz, 1H)
4,29 (q, J = 7,1 Hz, 2H)
4,02 (d, $J_{3,5}$ = 1,3 Hz, 2H)
1,33 (t, J = 7,1 Hz, 3H)

Beispiel 2

Analog Beispiel 1, nur anstatt mit Chlorameisensäureethylester mit 8,50 g (0,09 Mol) Chlorameisensäuremethylester, wurde in einer Ausbeute von 13,8 g = 79,2%, bezogen auf eingesetztes Oxetanon, das 4-(Methoxycarbonyloxy)-thiophen-2(5H)-on erhalten.

Das beige gefärbte Produkt hatte einen Schmelzbereich von 64–66°C und einen Gehalt nach GC von 92,1%.

$^1$H-NMR-Spektrum ($CDCl_3$, 300 MHz)
δ = 6,49 (t, $J_{3,5}$ = 1,3 Hz, 1H)
4,05 (q, $J_{3,5}$ = 1,3 Hz, 2H)
3,96 (s, 3H)

Beispiel 3

Eine Lösung von 16,3 g (0,1 Mol) 4-Chlor-4-chlormethyloxetan-2-on in 300 ml Tetrahydrofuran wurde auf –20°C abgekühlt und mit gasförmigem Schwefelwasserstoff gesättigt. Anschliessend gab man tropfenweise unter kräftigem Rühren innerhalb einer Stunde 20,4 g (0,2 Mol) Triethylamin zu. Man liess die Reaktionslösung auf Raumtemperatur erwärmen, rührte eine Stunde nach, filtrierte vom ausgefallenen Salz ab und dampfte das Lösungsmittel am Rotationsverdampfer zur Hälfte ab. Diese Lösung wurde mit 19,1 g (0,1 Mol) p-Toluolsulfonsäurechlorid versetzt, auf 0°C abgekühlt und tropfenweise mit 10,2 g (0,1 Mol) Triethylamin versetzt. Man filtrierte das ausgefallene Salz ab und engte die Reaktionslösung am Rotationsverdampfer ein. Der Rückstand wurde mit 50 ml Methanol gewaschen.

Man erhielt 19,8 g des 4-(p-Toluolsulfonyloxy)-thiophen-2(5H)-on als DC-reines Produkt vom Smp. 139–140°C.

Dies entsprach einer Ausbeute von 73,3%, bezogen auf eingesetztes Oxetanon.

$^1$H-NMR-Spektrum (300 MHz, $CDCl_3$)
δ = 7,86 (d, J = 8,8 Hz, 2H)
7,43 (d, J = 8,8 Hz, 2H)
6,13 (t, J = 1,2 Hz, 1H)
3,96 (d, J = 1,2 Hz, 1H)
2,50 (s, 3H)

Beispiel 4

Analog Beispiel 1, jedoch anstatt mit Chlorameisensäureethylester mit 12,3 g (0,09 Mol) Chlorameisensäurebutylester, wurde in einer Ausbeute von 75,7 %, bezogen auf eingesetztes Oxetanon, das 4-(n-Butyloxycarbonyloxy)-thiopen-2(5H)-on in Form schwach bräunlich gefärbter Kristalle von Schmelzpunkt 28–29°C erhalten.

$^1$H-NMR-Spektrum ($CDCl_3$, 300 MHz)
δ = 6,48 (t, $J_{3,5}$ = 1,2 Hz, 1H)
4,30 (t, J = 6,9 Hz, 2H)
4,09 (d, $J_{3,5}$ = 1,2 Hz, 2H)
1,73 (m, 2H)
1,44 (m, 2H)
0, 98 (t, J = 7,2 Hz, 3H)

Beispiel 5

Analog Beispiel 1, jedoch anstatt nur Chlorameisensäureethylester mit 15,3 g (0,09 Mol) Chlorameisensäurebenzylester, wurde in einer Ausbeute von 74,1%, bezogen auf eingesetztes Oxetanon, das 4-(Benzyloxycarbonyloxy)-thiophen-2(5H)-on in Form beige gefärbter Kristalle vom Schmelzpunkt 82–83°C erhalten.

$^1$H-NMR-Spektrum ($CDCl_3$, 300 MHz)
δ = 7,41 (s, 5H)
6,49 (t, $J_{3,5}$ = 1,2 Hz, 1H)
5,28 (s, 2H)
4,07 (d, $J_{3,5}$ = 1,2 Hz, 2H)

Beispiel 6

3,8 g (0,02 Mol) 4-(Ethoxycarbonyloxy)-thiophen-2(5H)-on wurden in 25 ml Tetrahydrofuran gelöst und auf –10°C abgekühlt. Dazu gab man eine Lösung von 2,9 g (0,04 Mol) Diethylamin in 35 ml Tetrahydrofuran. Man nutschte den ausgefallenen Feststoff ab (Diethylammoniumsalz der Thiotetronsäure) und nahm diesen in 20 ml Wasser auf. Unter Rühren wurde mit konz. Salzsäure auf pH 1–2 angesäuert.

Die ausgefallene Thiotetronsäure wurde mit 100 ml Essigester extrahiert. Die organische Phase wurde über $Na_2SO_4$ getrocknet und anschliessend das Lösungsmittel am Rotavapor abgedampft. Der Rückstand wurde am Hochvakuum getrocknet.

Man erhielt 2,0 g Thiotetronsäure als nahezu weisses, mikrokristallines Produkt vom Smp. 120–

122°C mit einem Gehalt von 96,9% (NaOH-Titration).

Dies entsprach 1,9 g 100%igem Produkt bzw. einer Ausbeute von 83,6%, bezogen auf eingesetztes 4-(Ethoxycarbonyloxy)-thiophen-2(5H)-on.

## Patentansprüche

1. Thiophenonderivate der allgemeinen Formel

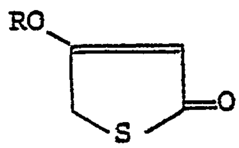

worin R eine $C_1$–$C_8$-Alkoxycarobnylgruppe, die unsubstituiert oder mit ($C_1$–$C_4$)-Alkylgruppe(n), Halogen-($C_1$–$C_4$)-alkylgruppe(n) oder Halogenatom(en) substituiert ist, eine Benzyloxycarbonylgruppe, die unsubstituiert oder mit Halogenatom(en), Nitrogruppe(n) oder ($C_1$–$C_4$)-Alkylgruppe(n) substituiert ist, oder eine p-Toluolsulfonylgruppe bedeutet.

2. 4-(Ethoxycarbonyloxy)-thiophen-2(5H)-on der Formel

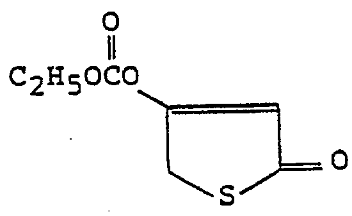

3. 4-(Methoxycarbonyloxy)-thiophen-2(5H)-on der Formel

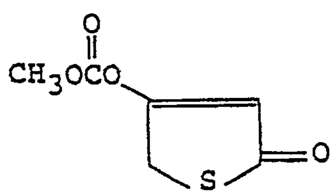

4. Verfahren zur Herstellung von Thiophenonderivaten nach Ansprüchen 1 bis 3, dadurch gekennzeichnet, daß 4-Chlor-4-chlormethyloxetan-2-on mit Schwefelwasserstoff in Gegenwart einer Base zur intermediär entstehenden Thiotetronsäure und diese anschließend mit einem Chlorameisensäureester der allgemeinen Formel ClCOOR, in der R die in Anspruch 1 genannte Bedeutung besitzt, oder einem p-Toluolsulfonsäurechlorid zum Endprodukt umgesetzt wird.

5. Verfahren nach Anspruch 4, dadurch gekennzeichnet, daß als Basen primäre, sekundäre oder tertiäre Amine, Ammoniak oder Guanidin angewendet werden.

6. Verfahren nach Ansprüchen 4 und 5, dadurch gekennzeichnet, daß als Basen tertiäre Amine angewendet werden.

7. Verfahren nach Ansprüchen 4 bis 6, dadurch gekennzeichnet, daß die Reaktion in inerten Lösungsmitteln durchgeführt wird.

8. Verfahren nach Ansprüchen 4 bis 7, dadurch gekennzeichnet, daß das Edukt-Molverhältnis Chlorameisensäureester/p-Toluolsulfonylchlorid zu Amin zu 4-Chlor-4-chlormethyloxetan-2-on 0,8:2,8:1,0 bis 1,0:4,0:1,0 beträgt.

9. Verfahren nach Ansprüchen 4 bis 8, dadurch gekennzeichnet, daß das Edukt-Molverhältnis von Schwefelwasserstoff zu 4-Chlor-4-chlormethyloxetan-2-on 1:1 bis 1:3 beträgt.

10. Verfahren nach Ansprüchen 4 bis 7, dadurch gekennzeichnet, daß in einem Temperaturbereich zwischen –40 bis +20°C gearbeitet wird.

11. Verwendung der Thiophenonderivate gemäß Anspruch 1 zur Herstellung von Thiotetronsäure.

## Claims

1. Thiophenone derivatives of the general formula

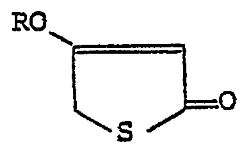

wherein R is a $C_1$–$C_8$-alkoxy carbonyl group, which is unsubstituted or substituted with $C_1$–$C_4$-alkyl group(s), halogen-($C_1$–$C_4$)-alkyl group(s) or halogen atom(s), a benzyloxycarbonyl group, which is unsubstituted or substituted with halogen atom(s), nitro group(s) or ($C_1$–$C_4$)-alkyl group(s), or a p-toluene sulfonyl group.

2. 4-(Ethoxycarbonyloxy)-thiophene-2(5H)-one of the formula

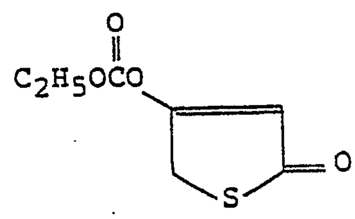

3. 4-(Methoxycarbonyloxy)-thiophene-2(5H)-one of the formula

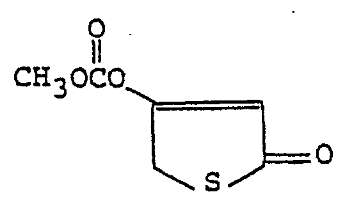

4. Process for the preparation of thiophenone derivatives according to claims 1 to 3, characterized in that 4-chloro-4-chloromethyloxetane-2-one is reacted with hydrogen sulfide in the presence of a base obtaining thiotetronic acid as intermediate and that the latter is subsequently reacted with a

chloroformic acid ester of the general formula Cl-COOR, in which R has the meaning mentioned in claim 1, or a p-toluene sulfonic acid chloride obtaining the end product.

5. Process according to claim 4, characterized in that as bases primary, secondary or tertiary amines, ammonia or guanidine are used.

6. Process according to claims 4 and 5, characterized in that as bases tertiary amines are used.

7. Process according to claims 4 to 6, characterized in that the reaction is carried out in inert solvents.

8. Process according to claims 4 to 7, characterized in that the molar educt ration of chloroformic acid ester/p-toluene sulfonyl chloride to amine to 4-chloro-4-chloro-methyloxetane-2-one is 0.8:2.8:1.0 to 1.0:4.0:1.0.

9. Process according to claims 4 to 8, characterized in that the molar educt ratio of hydrogen sulfide to 4-chloro-4-chloromethyloxetane-2-one is 1:1 to 1:3.

10. Process according to claims 4 to 7, characterized in that one operates in a temperature range between –40 to +20°C.

11. Use of the thiophenone derivatives according to claim 1 for the preparation of thiotetronic acid.

**Revendications**

1. Dérivés de thiophénone de formule générale

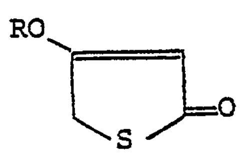

dans laquelle R représente un groupe alcoxycarbonyle en $C_1$–$C_8$, qui est non-substitué ou substiué par un ou des groupe(s) alcoyle en $C_1$–$C_4$, un ou des groupe(s) halogéno-alcoyle en $C_1$–$C_4$ ou un ou des atome(s) d'halogène, ou représente un groupe benzyloxycarbonyle qui est non substitué par un des atome(s) d'halogène, un ou des groupe(s) nitro ou un ou des groupe(s) alcoyle en $C_1$–$C_4$, ou représente un groupe p-toluènesulfonyle.

2. La 4-(éthoxycarbonyloxy)-thiophène-2(5H)-one de formule

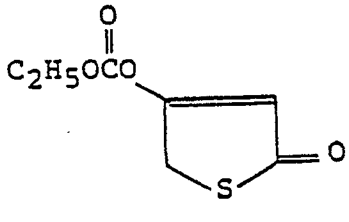

3. La 4-(méthoxycarbonyloxy)-thiophène-2(5H)-one de formule

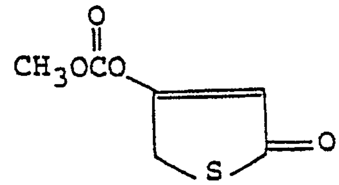

4. Procédé pour la préparation de dérivés de thiophénone selon les revendications 1 à 3, caractérisé en ce qu'on fait réagir la 4-chloro-4-chlorométhyloxétane-2-one avec de l'hydrogène sulfuré en présence d'une base pour donner l'acide thiotétronique résultant de façon intermédiaire et en ce qu'on fait réagir ensuite celui-ci avec un ester de l'acide chloroformique de formule générale Cl-COOR, dans laquelle R possède la signification mentionnée à la revendication 1, ou avec un chlorure de l'acide p-toluènesulfonique pour donner le produit final.

5. Procédé selon la revendication 4, caractérisé en ce qu'on utilise, comme bases des amines primaires, secondaires ou tertiaires, l'ammoniac ou la guanidine.

6. Procédé selon les revendications 4 et 5, caractérisé en ce qu'on utilise comme bases des amines tertiaires.

7. Procédé selon les revendications 4 à 6, caractérisé en ce que la réaction est effectuée dans des solvants inertes.

8. Procédé selon les revendications 4 à 7, caractérisé en ce que le rapport molaire des produits de départ ester de l'acide chloroformique/chlorure de l'acide p-toluènesulfonique à l'amine à la 4-chloro-4-chlorométhyloxétane-2-one est de 0,8:2,8:1,0 à 1,0:4,0:1,0.

9. Procédé selon les revendications 4 à 8, caractérisé en ce que le rapport molaire des produits de départ hydrogène sulfuré à 4-chloro-4-chlorométhyloxétane-2-one est de 1:1 à 1:3.

10. Procédé selon les revendications 4 à 7, caractérisé en ce que l'on travaille dans un domaine de températures entre –40 jusqu'à +20°C.

11. Utilisation des dérivés de thiophénone selon la revendication 1 pour la préparation de l'acide thiotétronique.